# EUROPEAN PATENT SPECIFICATION

(11) **EP 1 103 990 B1**
(45) Date of publication and mention of the grant of the patent: **09.05.2007**
(21) Application number: 00123852.6
(22) Date of filing: 02.11.2000
(51) Int. Cl.: H01F 7/02, A61N 2/06

(54) **Magnetizing container**
Magnetisierender Behälter
Récipient magnétisant

(30) Priority: 26.11.1999 IT MI990717 U
(43) Date of publication of application: 30.05.2001
(73) Proprietor: FI.MA.STARS S.r.l., 21022 Azzate (Varese) (IT)
(72) Inventor: Scapinello, Marino, 21040 Carnago (Varese) (IT)
(74) Representative: Lecce, Giovanni

(56) References cited:
- BE-A- 460 974
- DE-A- 3 629 761
- DE-A- 3 634 121
- PATENT ABSTRACTS OF JAPAN vol. 018, no. 542 (C-1261), 17 October 1994 (1994-10-17) & JP 06 190368 A (MAKOTO YAFUJI;OTHERS: 01), 12 July 1994 (1994-07-12)
- PATENT ABSTRACTS OF JAPAN vol. 015, no. 422 (M-1173), 25 October 1991 (1991-10-25) & JP 03 176381 A (RIBURAIFU KENKYUSHO:KK), 31 July 1991 (1991-07-31)

## Description

The present invention relates to a magnetizing container.

More particularly, the present invention relates to a container from glass, plastic materials or other suitable materials, provided with magnets suitable to magnetize the substances contained therein.

The substances contained in the container may be liquid, cream-like or gel-like, preferably creams, lotions, gels and fluid products of various type utilized for skin-treatments, as well as dietetic alimentary or nutritional foods.

As is known, different treatments carried out with magnets produce beneficial effects on human organisms. Studies and experiments carried out have demonstrated that the so-called natural magnetotherapy maintains the energetic balance of human body and accelerates also the regeneration of the tissues of the skin. For this purposes, magnets are traditionally used in several ways, with direct or indirect application on some body parts.

In case of indirect application, some magnets are utilized to magnetize liquid substances, drinks included, or as creams, lotions and gels to be applied on the skin for cosmetic purposes or to attenuate inflammatory conditions. The magnet is placed in the containers of these substances and therefore it is immersed in the substances.

The substance, in the form of a liquid, an oil, a cream, a gel and the like, is consequently magnetized, improving its optimal effect, in terms of absorption, once it is applied to the body.

However, this type of magnetization has a severe drawback, due essentially to the direct contact of the magnet with the substance contained in the container. The magnet, in fact, cannot be reused when the container is exhausted and it may be the involuntary cause of the pollution of the substances that, once applied to the body or ingested, can give rise to undesired reactions. In some cases, a chemical reaction can take place between the metal magnet and the fluids present in the container, with an ensuing development of toxic substances.

DE 36 29 761 discloses a package for liquid material comprising at least one permanent magnet with the magnetic field penetrating at least partially into the inner volume of the package to magnetise the content thereof.

DE 36 34 121 relates to a method for improving the effects of liquid or pasty products intended for use on human body skin, wherein these products are exposed before use to an equidirectional magnetic field. With reference to a pasty material, Fig. 2 shows a container with a screw lid, both incorporating a magnet.

Object of this invention is to obviate the drawbacks of the prior art devices.

More particularly, object of this invention is to realize a magnetizing container, especially for fluid substances, that allows to magnetize the substance contained in the container, without however altering its organoleptic characteristics and allowing an easy reutilization of the magnets.

A further object of the invention is to realize a magnetizing container suitable to ensure the unaltered maintaining - from the viewpoint of health and efficiency characteristics - of the substances or products contained therein.

A further object of the invention is to put at the disposal of users a magnetizing container suitable to ensure a high level of reliability in the time, and such also as to be easily and economically realized.

According to the present invention, these and still other objects that will become apparent from the following description are achieved by a magnetizing container as claimed in claim 1. Additional advantageous features are recited in the dependent claims.

Said container is obtainable from plastic material, glass or other suitable materials.

The characteristic of the present invention will be better understood thanks to the following description, wherein reference is made to the attached drawings that represent a simplified preferred non-limiting embodiment, and wherein:

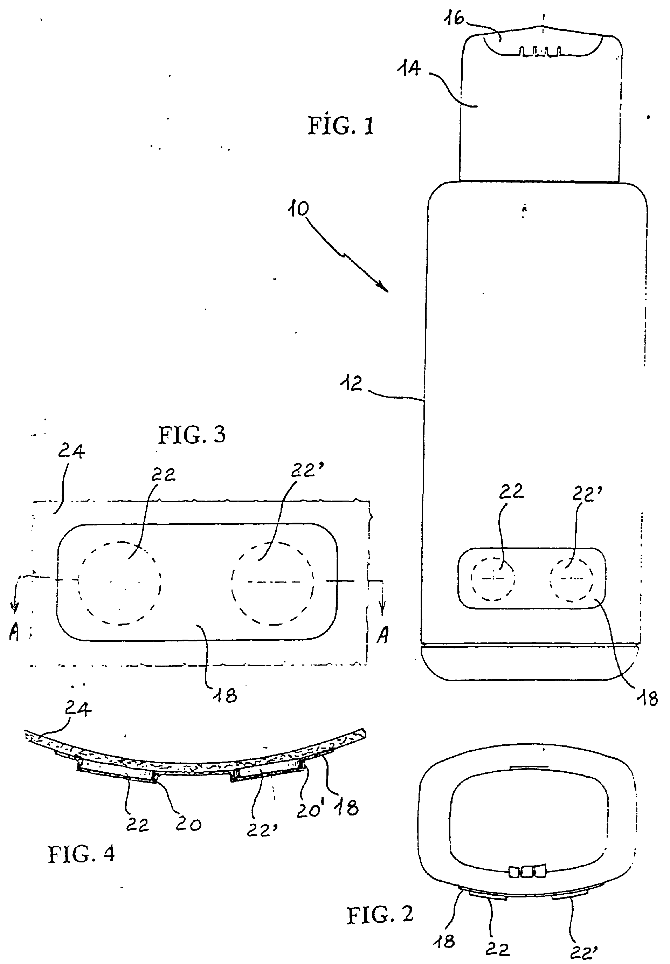

Figure 1 is a front schematic view of the magnetizing container of the invention;
Figure 2 is a top schematic view of the same container of Figure 1;
Figure 3 is the schematic view of a magnified detail of the container of the preceding figures, in its part provided with means suitable to magnetize its contents;
Figure 4 is the schematic view of a section along the A - A line of the container of Figure 3 and the related magnetizing means.

With reference to the aforesaid figures, the magnetizing container of the present invention, indicated by 10 as a whole, is made, by way of example, from plastic material, glass or other suitable materials, of any form and size, plugged by a plug or capsule 14.

Said plug or capsule 14 is preferably provided with a movable or tiltable part 16, formed on the top or the sides, which forms a tight-screen that can be opened to extract, when necessary, from flacon 12 a part of its contents.

According to the embodiment illustrated in the figures, said flacon 12 is constituted of a body having a substantially rectangular plan, with opposite convex major sides and a height markedly greater than the base size. Such configuration of the container or flacon 12 is merely indicative, as well as the shape of its profile; obviously, therefore, such container or flacon may take on variable shapes, sizes and configurations.

According to the present invention, an envelope 18 is applied to one 24 of the external side surface of flacon 12, said envelope having, by way of example, a rectangular shape and being obtainable from flexible plastic material preferably having a constant and limited thickness. Said envelope is so shaped as to define, along its surface, at least a seat, that may have a circular plan or other shapes, complementary to the one of a magnet.

Envelope 18, which forms substantially a blister, preferably integrates two coupled seats 20, 20' for as many magnets or magnetizing bodies 22, 22'. Said magnets or magnetizing bodies 22, 22' are located in said seats 20, 20', with an opposite orientation with respect to the side surface 24 of flacon 12. In detail, one of the magnets, for instance magnet 22, is positioned, with respect to surface 24, with its "N" polarity, while the second adjoining magnet 22' directs its "S" polarity towards said surface. This causes the magnetic field to close with regard to the substance contained in flacon 12 and its complete magnetization.

Instead of the two magnetic bodies 22, 22' it is possible to use one only magnet with both polarities on the same surface or face of the container along differentiated zones.

Envelope 18 in its non-exposed part intended for coming in touch with the external side surface 24 of flacon 12, is provided with an adhesive layer compatible with the material forming said flacon. Alternatively or additionally to said adhesive layer, envelope 18 incorporating magnets 22, 22' may be tied to the external side surface 24 of flacon 12, in any position, through the inclusion of an adhesive strip, possibly holed in correspondence of the strike-zones with seats 20, 21' which remain therefore exposed.

In the exemplary embodiment of the figures, magnets 22, 22' have a cylindrical configuration with a reduced height, but the same may be obviously constituted of metal bodies of any form and size from ferromagnetic material.

According to another alternative embodiment, container 10 may be provided along its external side surface with hollow seats or impressions to house magnets 22, 22'. Said seats, that need not being aligned or adjoining with each other, are circumscribed towards the outside with an adhesive strip or the like, that locks said magnets in their position.

The magnets or magnetized bodies 22, 22' may be located in a different position with respect to the external side surface, for instance integrated or connected with the external surface of the bottom, the plug or the closing capsule in correspondence of its external and/or internal side surface.

As can be inferred from the above description, the advantages achieved by the magnetizing container of the present invention are evident.

It allows to conveniently magnetize the substances contained therein, avoiding the problems arising from the contact between the magnet and the substances.

The simultaneous presence of two magnets, oriented with opposite polarities with respect to the container, causes the full closing of the magnetic field, optimizing the effect induced by the substances.

A further advantage lies in that the container of the present invention allows the substantially immediate recovery and utilization of the magnetic body or bodies, and their possible reutilization in conditions of full integrity and effectiveness.

The magnets may be re-utilized by applying envelope 18 to another flacon, once the contents of the first one is used up, said magnets being perfectly integer, not soiled or conditioned by the product.

Besides, with the magnetizing container of the present invention, it is extremely easy to remove the magnets from the container for the differentiated waste collection and subsequent dumping.

While the present invention has been described above with reference to an embodiment, it is obvious that many alternatives and variants may be introduced by those skilled in the art, in the light of the above description. Therefore, the present invention intends to comprise all the alternatives and variants that fall within the scope of the following claims.

## Claims

1. A magnetizing container (10), especially suitable for fluids, obtainable from plastic, glass or other suitable materials, provided with at least one magnet (22, 22') adapted to magnetize the substances contained in the container, said at least one magnet (22, 22') being connected or integrated with an external surface of the container or components thereof, and said at least one magnet having both polarities located on a same surface face of the container along different zones so that the permanent magnetic field penetrates at least partially into said container (10), **characterized in that** it comprises an envelope (18), made from flexible plastic material or other suitable materials, provided with at least one shaped recess (20, 21') for receiving said at least one magnet (22, 22') said envelope is made tacky on the front surface and contacts the external surface of said container (12).

2. The container according to claim 1, **characterized in that** it comprises at least a couple of magnetic bodies (22, 22') connected or integrated with an external surface of the container and having polarities located on the same surface or face of the container, along differentiated zones.

3. The container according to claim 2, **characterized in that** said magnetic bodies or magnets (22, 22') are located and oriented according to polarities opposite with respect to the external side surface (24) of container (12).

4. The container according to claim 3, **characterized in that** one (22) of said magnets is positioned, with respect to surface (24), with its "N" polarity, and the second adjoining magnet (22') directs its "S" polarity towards said surface (24).

5. The container according to claims 1 to 4, **characterized in that** said envelope (18) is partly surmounted by an adhesive strip for the connection with the container or components thereof.

6. The container according to claim 5, **characterized in that** said connection strip is provided with holes in correspondence of the recesses (20, 21').

7. The container according to any of the preceding claims, **characterized in that** it is provided along the external side surface with two hollow seats or impressions for housing said magnets (22, 22'), said magnets being surmounted on the exposed front by an adhesive strip.

8. The container according to any of the preceding claims, **characterized in that** said magnets (22, 22') are located along the external and/or internal side surface of a plug (14).

9. The container according to any of the preceding claims 1-8, **characterized in that** said magnets (22, 22') are located on the external surface of the container bottom.

10. The container according to any of the preceding claims 1-8, **characterized in that** magnets (22, 22') are located on the external surface of the container bottom.

## Patentansprüche

1. Magnetisierender Behälter (10), insbesondere geeignet für Fluide, der aus Kunststoff, Glas oder einem anderen geeigneten Material bestehen kann und mit wenigstens einem Magnet (22, 22') versehen ist, der geeignet ist, die in dem Behälter befindlichen Substanzen zu magnetisieren, wobei dieser wenigstens eine Magnet (22, 22') mit einer Außenfläche des Behälters oder einem Element von diesem verbunden oder einstückig damit ausgebildet ist und dieser wenigstens eine Magnet beide Polaritäten auf der gleichen Oberflächenseite des Behälters längs unterschiedlicher Zonen aufweist, sodass das Permanentmagnetfeld wenigstens teilweise in den Container (10) eindringt,
**dadurch gekennzeichnet,**
**dass** eine Umhüllung (18) aus einem flexiblen Kunststoffmaterial oder einem anderen geeigneten Material mit wenigstens einer geformten Ausnehmung (20, 20') zur Aufnahme dieses wenigstens einen Magneten (22, 22') vorgesehen ist, wobei diese Umhüllung auf der Vorderseite haftet und mit der Außenseite des Behältere (12) in Kontakt ist.

2. Behälter nach Anspruch 1, **dadurch gekennzeichnet, dass** wenigstens ein Paar von Magnetkörpern (22, 22') vorgesehen ist, die mit einer Außenfläche des Behälters verbunden sind oder aus einem Stück mit diesem bestehen sowie Polaritäten aufweisen, die auf der gleichen Fläche oder Seite des Behälters längs unterschiedlicher Zonen liegen.

3. Behälter nach Anspruch 2, **dadurch gekennzeichnet, dass** die Magnetkörper oder Magneten (22, 22') hinsichtlich Polaritäten gegenüberliegend zu der Außenseitenfläche (24) des Behälters (12) angeordnet und ausgerichtet sind.

4. Behälter nach Anspruch 3, **dadurch gekennzeichnet, dass** einer (22) der Magneten hinsichtlich der Oberfläche (24) mit seiner "N" Polarität angeordnet ist und der zweite benachbarte Magnet (22') seine "S" Polarität in Richtung auf diese Fläche (24) richtet.

5. Behälter nach den Ansprüchen 1 - 4, **dadurch gekennzeichnet, dass** die Umhüllung (18) teilweise durch einen Klebestreifen zur Verbindung mit dem Behälter oder einem Element von diesem angebracht ist.

6. Behälter nach Anspruch 5, **dadurch gekennzeichnet, dass** der Verbindungsstreifen mit Löchern in Übereinstimmung mit den Ausnehmungen (20, 20') versehen ist.

7. Behälter nach einem der vorhergehenden Ansprüche, **dadurch gekennzeichnet, dass** er längs der Außenseite mit zwei hohlen Sitzen oder Einprägungen zur Aufnahme der Magnete (22, 22') versehen ist, wobei die Magnete auf der freiliegenden Frontseite durch einen Klebestreifen überdeckt sind.

8. Behälter nach einem der vorhergehenden Ansprüche, **dadurch gekennzeichnet, dass** die Magneten (22, 22') längs einer äußeren und/oder inneren Seite eines Stöpsels (14) angeordnet sind.

9. Behälter nach einem der vorhergehenden Ansprüche 1 - 8, **dadurch gekennzeichnet, dass** die Magneten (22, 22') auf der Außenfläche des Behälterbodens angeordnet sind.

## Revendications

1. Récipient magnétisant (10), spécialement adapté à des fluides, réalisable en matière plastique, en verre ou en d'autres matières appropriées, comportant au moins un aimant (22, 22'), apte à magnétiser les substances contenues dans le récipient, ledit au moins un aimant (22, 22') étant connecté à la surface externe ou à des composants du récipient, ou étant intégré avec celle-ci ou ceux-ci, et les deux polarités dudit au moins un aimant étant situées sur une même face de surface du récipient dans des zones différentes, afin que le champ magnétique permanent pénètre au moins partiellement dans ledit récipient (10), **caractérisé en ce qu'**il comprend une enveloppe (18) en matière plastique flexible ou en autres matières appropriées, dans laquelle est ménagé au moins un évidement (20, 20') configuré pour recevoir ledit au moins un aimant (22, 22'), et que ladite enveloppe est rendue collante sur la surface frontale et est en contact avec la surface externe dudit récipient (12).

2. Récipient selon la revendication 1, **caractérisé en ce qu'**il comprend au moins un couple de corps magnétiques (22, 22'), qui sont connectés à une surface externe du récipient ou intégrés dans celle-ci, et dont les polarités sont situées sur la même surface ou face du récipient, le long de zones différenciées.

3. Récipient selon la revendication 2, **caractérisé en ce que** lesdits corps magnétiques ou aimants (22, 22') sont situés et orientés selon des polarités opposées par rapport à la surface (24) située sur le côté externe du récipient (12).

4. Récipient selon la revendication 3, **caractérisé en ce que** l'un (22) desdits aimants est positionné pour que sa polarité "N" soit tournée vers la surface (24) et que le deuxième aimant voisin (22') dirige sa polarité "S" vers ladite surface (24).

5. Récipient selon l'une quelconque des revendications précédentes, **caractérisé en ce que** ladite enveloppe (18) est partiellement surmontée par une bande adhésive pour la connexion avec le récipient ou des composants de celui-ci.

6. Récipient selon la revendication 5, **caractérisé en ce que** des orifices sont ménagés dans ladite bande de connexion en correspondance avec les évidement (20, 20').

7. Récipient selon l'une quelconque des revendications précédentes, **caractérisé en ce que** deux logements ou empreintes creux sont ménagés le long de la surface située sur le côté externe pour loger lesdits aimants (22, 22'), lesdits aimants étant surmontés par une bande adhésive sur la partie frontale exposée.

8. Récipient selon l'une quelconque des revendications précédentes, **caractérisé en ce que** lesdits aimants (22, 22') sont situés le long de la surface située sur le côté externe et/ou interne d'un bouchon (14).

9. Récipient selon l'une quelconque des revendications précédentes, **caractérisé en ce que** lesdits aimants (22, 22') sont situés sur la surface externe du bas du récipient.
